# EUROPEAN PATENT SPECIFICATION

(11) **EP 1 765 809 B1**
(45) Date of publication and mention of the grant of the patent: **31.12.2008**
(21) Application number: 04743765.2
(22) Date of filing: 16.06.2004
(51) Int. Cl.: C07D 405/12, A61K 31/40, A61P 11/00, A61P 1/00

(54) **XANTHINE DERIVATIVES USEFUL AS MUSCARINIC RECEPTOR ANTAGONISTS**
ALS ANTAGONISTEN DES MUSCARINREZEPTORS GEEIGNETE XANTHINDERIVATE
DERIVES DE XANTHINE UTILISES EN TANT QU'ANTAGONISTES DU RECEPTEUR MUSCARINIQUE

(43) Date of publication of application: 28.03.2007
(73) Proprietor: Ranbaxy Laboratories Limited, Gurgaon, Haryana 122001 (IN)
(72) Inventor: MEHTA, Anita, Plainfield, Illinois 60586 (US); SALMAN, Mohammad, Princeton, NJ 08540 (US); SARMA, Pakala, Kumara, Savithru, Gurgaon, Haryana 122001 (IN); CHUGH, Anita, New Delhi, Delhi 110012 (IN); GUPTA, Suman, Gurgaon, Haryana 122001 (IN)
(74) Representative: Cronin, Brian Harold John
(86) International application number: PCT/IB2004/002004
(87) International publication number: WO 2006/005980

(56) References cited:
- EP-A- 0 309 422
- WO-A-01/04118
- WO-A-98/05292
- WO-A-20/04056810

## Description

### Field of the Invention

This present invention generally relates to xanthine derivatives as muscarinic receptor antagonists which are useful, among other uses, for the treatment of various diseases of the respiratory, urinary and gastrointestinal systems mediated through muscarinic receptors. The invention also relates to the process for the preparation of disclosed compounds and pharmaceutical compositions containing the disclosed compounds.

### Background of the Invention

Muscarinic receptors as members of the G Protein Coupled Receptors (GPCRs) are composed of a family of 5 receptor sub-types (M₁, M₂, M₃, M₄ and M₅) and are activated by the neurotransmitter acetylcholine. These receptors are widely distributed on multiple organs and tissues and are critical to the maintenance of central and peripheral cholinergic neurotransmission. The regional distribution of these receptor sub-types in the brain and other organs has been documented. (for example, the M₁ subtype is located primarily in neuronal tissues (for example, cereberal cortex and autonomic ganglia, the M₂ subtype is present mainly in the heart where it mediates cholinergically induced bradycardia, and the M₃ subtype is located predominantly on smooth muscle and salivary glands (Nature, 323, p.411 (1986); Science, 237, p.527 (1987)).

A review in Current Opinions in Chemical Biology, 3, p. 426 (1999), as well as in Trends in Pharmacological Sciences, 22, p. 409 (2001) by Eglen et. al., describes the biological potentials of modulating muscarinic receptor subtypes by ligands in different disease conditions (for example, Alzheimer's Disease, pain, urinary disease condition, chronic obstructive pulmonary disease, and the like).

Muscarinic agonists (for example, muscarine and pilocarpine and antagonists (for example, atropine have been known for over a century, but little progress has been made in the discovery of receptor subtype-selective compounds, making it difficult to assign specific functions to the individual receptors. Although classical muscarinic antagonists (for example, atropine) are potent bronchodilators, their clinical utility is limited due to high incidence of both peripheral and central adverse effects (for example, tachycardia, blurred vision, dryness of mouth, constipation, dementia, etc.). Derivatives of atropine (for example, ipratropium bromide) are better tolerated than parenterally administered options, but most of these are not ideal anti-cholinergic bronchodilators, due to lack of selectivity for muscarinic receptor sub-types, resulting in dose-limiting side-effects (for example, thirst, nausea, mydriasis and those associated with the heart, for example, tachycardia) mediated by the M₂ receptor.

Annual Review of pharmacological Toxicol., 4 1, p. 691 (2001), describes the pharmacology of the lower urinary tract infections. Although anti-muscarinic agents (for example, oxybutynin and tolterodine that act non-selectively on muscarinic receptors have been used for many years to treat bladder hyperactivity, the clinical effectiveness of these agents has been limited due to the side effects (for example, dry mouth, blurred vision and constipation). Tolterodine is considered to be generally better tolerated than oxybutynin. (Steers et. al., in Curr. Opin. Invest. Drugs, 2, 268; Chapple et. al., in Urology, 55, 33; Steers et al., Adult and Pediatric Urology, ed. Gillenwatteret al., pp 1220-1325, St. Louis, MO; Mosby. 3rd edition (1996)).

There remains a need for development of new highly selective muscarinic antagonists which can interact with distinct subtypes, thus avoiding the occurrence of adverse effects.

Compounds having antagonistic activity against muscarinic receptors have been described in Japanese patent application Laid Open Number 92921/1994 and 135958/1994; WO 93/16048; U.S. Patent No. 3,176,019; GB 940,540; EP 0325 571; WO 98/29402; EP 0801067; EP 0388054; WO 9109013; U.S. Patent No. 5,281,601. Also, U.S. Patent Nos. 6,174,900, 6,130,232 and 5,948,792; WO 97/45414 are related to 1,4-disubstituted piperidine derivatives; WO 98/05641 describes fluorinated, 1,4-disubstitued piperidine derivatives; WO 93/16018 and WO96/33973 are other references of interest. US Patent No. 5,397,800 discloses 1-azabicyclo[2.2.1]heptanes. US Patent No.5, 001,160 describes 1-aryl-1-hydroxy-1-substituted-3-(4-substituted-1-piperazinyl)-2-propanones. WO 01/42213 describes 2-biphenyl-4-piperidinyl ureas. WO 01/42212 describes carbamate derivatives. WO 01/90081 describes amino alkyl lactam. WO 02/53564 describes novel quinuclidine derivatives WO 02/00652 describes carbamates derived from arylalkyl amines. WO 02/06241 describes 1,2,3,5-tetrahydrobenzo(c)azepin-4-one derivatives. U.S. application No. 20030105071 describes thiazole and other heterocyclic ligands for mammalian dopamine, muscarinic and serotonic receptors and transporters, and method of use thereof. WO 04/005252 discloses azabicyclo derivatives as musacrinic receptor antagonists. WO04014853, WO04014363 and WO 04/004629 discloses 3,6-disubstituted azabicyclo [3.1.0] hexane derivatives useful as muscarinic receptor antagonists.

J.Med Chem., 44, p. 984 (2002), describes cyclohexylmethylpiperidinyl-triphenylpropioamide derivatives as selective M₃ antagonist discriminating against the other receptor subtypes. J.Med. Chem., 36, p. 610 (1993), describes the synthesis and antimuscarinic activity of some 1-cycloalkyl-1-hydroxy-1-phenyl-3-(4-substituted piperazinyl)-2-propanones and related compounds. J.Med. Chem., 34, p.3065 (1991), describes analogues of oxybutynin, synthesis and antimuscarinic activity of some substituted 7-amino-1-hydroxy-5-heptyn-2-ones and related compounds.

EP-A-0 309 422 discloses amidino tricycle derivatives as muscarinic receptor blocking agents, said to be useful for the treatment of gastrointestinal disorders. Processes for their preparation and pharmaceutical compositions containing them are also discussed therein.

WO-A-01/04118 discloses quinuclidine derivatives and medicinal compositions containing such compounds. These compounds are described as having high affinity for muscarinic M3 receptors, potentially useful against particular respiratory, urological and gastrointestinal disorders.

WO-A-2004/056810 discloses xanthine derivatives as muscarinic receptor antagonists. The compounds can generally be described as 3,6-disubstituted azabicyclo[3.1.0] hexanes, and are useful for the treatment of respiratory, urinary and gastrointestinal systems mediated through muscarinic receptors.

### Summary of the Invention

In one aspect, there are provided xanthine derivatives as muscarinic receptor antagonists, which can be useful as safe and effective therapeutic or prophylactic agents for the treatment of various diseases of the respiratory, urinary and gastrointestinal systems. Also provided are processes for synthesizing such compounds.

In another aspect, pharmaceutical compositions containing such compounds arc provided together with acceptable carriers, excipients or diluents which can be useful for the treatment of various diseases of the respiratory, urinary and gastrointestinal systems.

The enantiomers, diastereomers, N-oxides, polymorphs, pharmaceutically acceptable salts and pharmaceutically acceptable solvates of these compounds are also provided, as well as pharmaceutical compositions comprising the compounds, their enantiomers, diastereomers, N-oxides or polymorphs or pharmaceutically acceptable salts thereof, in combination with a pharmaceutically acceptable carrier and optionally included excipients.

Other aspects will be set forth in the description which follows, and in part will be apparent from the description or may be learnt by the practice of the invention.

In accordance with one aspect, there are provided the compounds listed in claim 1, having the structure of Formula I: 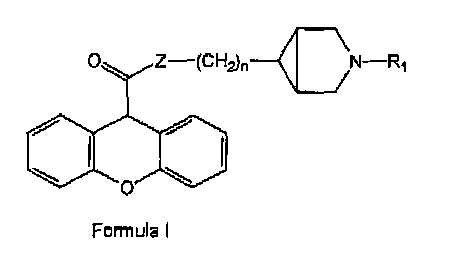 and their pharmaceutically acceptable salts, pharmaceutically acceptable solvates, enantiomers, diastereomers, N-oxides or polymorphs. In Formula I
Z is oxygen, or
-NRₓ wherein Rₓ is selected from hydrogen, lower (C₁₋₆) alkyl, or aralkyl.
n is an integer from 0-4.
R₁ is hydrogen, alkyl optionally substituted with aryl or heteroaryl, or alkenyl.

In accordance with a second aspect, there is provided a pharmaceutical composition comprising therapeutically effective amount of a compound as defined in claim 1 together with pharmaceutically acceptable carrier, excipients or diluents.

In accordance with a third aspect, there is provided a compound as defined in claim 1 or a pharmaceutical composition as just defined, for a treatment or prophylaxis of an animal or a human suffering from a disease or disorder of the respiratory, urinary and gastrointestinal systems, wherein the disease or disorder is mediated through muscarinic receptors.

In accordance with a fourth aspect, there is provided a compound as defined in claim 1 or a pharmaceutical composition as defined above, for treatment or prophylaxis of an animal or a human suffering from a disease or disorder of the respiratory system (for example, bronchial asthma, chronic obstructive pulmonary disorders (COPD), pulmonary fibrosis, and the like; urinary system which induce such urinary disorders as urinary incontinence, lower urinary tract symptoms (LUTS), etc.; and gastrointestinal system (for example, irritable bowel syndrome, obesity, diabetes and gastrointestinal hyperkinesis, wherein the disease or disorder is associated with muscarinic receptors.

In accordance with a fifth aspect, there are provided processes for preparing the compounds as described above.

The compounds described herein exhibit significant potency in terms of their activity, as determined by *in vitro* receptor binding and functional assays and *in vivo* experiments using anaesthetized rabbits. The compounds that were found active *in vitro* were tested *in vivo.* Some of the compounds are potent muscarinic receptor antagonists with high affinity towards M₃ receptors. Therefore, pharmaceutical compositions for the possible treatment for the disease or disorders associated with muscarinic receptors are provided. In addition, the compounds can be administered orally or parenterally.

### Detailed Description of the Invention

The compounds of the present invention may be prepared by methods represented by the reaction sequences as shown in Schemes I, II and III:

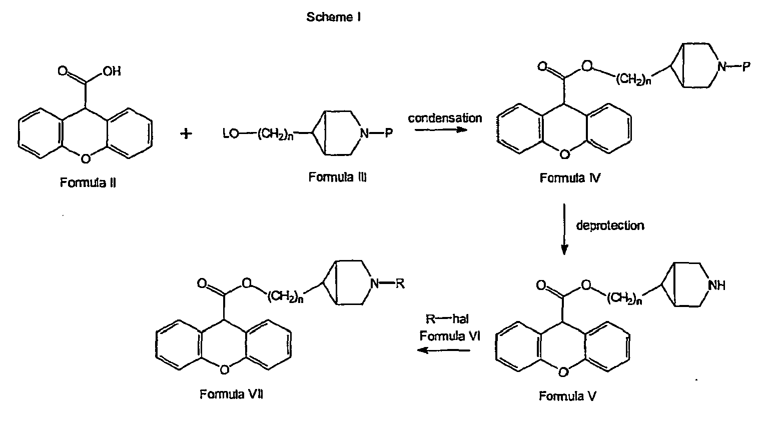

The compound of Formula VII may be prepared, for example, by the reaction sequence as shown in Scheme I. The prepration comprises condensing a compound of Formula II with a compound of Formula III (wherein L is a leaving group for example, mesyl or tosyl, n is same as defined earlier and P is a protecting group for example, aralkyl) to give a compound of Formula IV, which is deprotected to give a compound of Formula V, which is reacted with a compound of Formula VI (wherein R is heteroarylalkyl or alkenyl to give a compound of Formula VII.

The compound of Formula II can be condensed with a compound of Formula III in an organic solvent (for example, toluene, xylene or benzene) with a condensing agent (for example, 1,8-diazabicyclo[5.4.0]undecen-7-ene or 1,4-diazabicyclo[2.2.2]octane) to give a compound of Formula IV, which can be deprotected in an organic solvent (for example, methanol, ethanol, propanol, isopropylalcohol, tetrahydrofuran or ethyl acetate) under the condition of deprotection (for example, hydrogenatically utilizing palladium on carbon or under catalytic transfer hydrogen conditions of ammonium formate and palladium on carbon) to give a compound of Formula V which can be reacted with a compound of Formula VI in an organic solvent (for example, acetonitrile, dimethylsulphoxide or dimethylformamide) in the presence of base (for example, potassium carbonate, sodium carbonate or sodium bicarbonate) to give a compound of Formula VII.

Particular compounds according to the invention are shown here:
9H-Xanthene-9-carboxylic acid [(1α, 5α, 6α)-3-benzyl-3-aza-bicyclo[3.1.0]hex-6-ylmethyl] ester (Compound No. 1);
9H-Xanthene-9-carboxylic acid [(1α, 5α, 6α)-3-aza-bicyclo[3.1.0]hex-6-ylmethyl] ester (Compound No. 5);
9H-Xanthene-9-carboxylic acid [(1α, 5α, 6α)-3-(4-methyl-pent-3-enyl)-3-azabicyclo[3.1.4]hex-6-ylmethyl] ester (Compound No. 10).

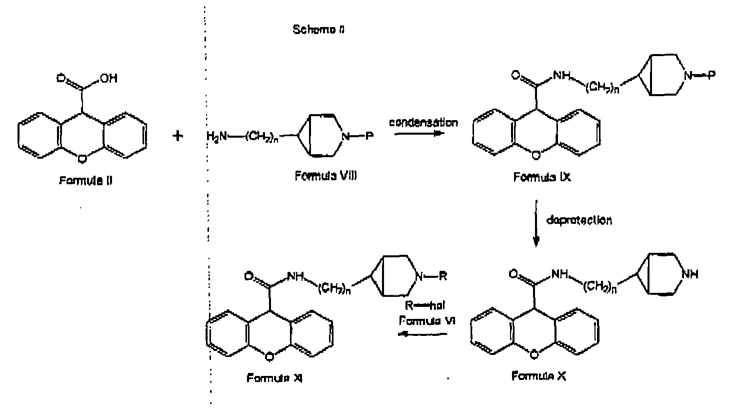

The compound of Formula XI, may be prepared by, for example, by the reaction sequence as shown in Scheme II. The prepration comprises condensing a compound of Formula II with a compound of Formula VIII (wherein P is a protecting group for example, aralkyl and n is same as defined earlier) to give a compound of Formula IX, which is deprotected to give a compound of Formula X, which is reacted with a compound of Formula VI [wherein R is heteroarylalkyl or alkenyl group and hal is a halogen (Cl, Br, I)] to give a compound of Formula XI.

The condensation of compound of Formula II with a compound of Formula VIII give a compounds of Formula IX can be carried out in an organic solvent (for example, chloroform or dimethylformamide) with a condensing agent (for example, 1-(3-dimethylaminopropyl)-3-ethylcarbodiimide hydrochloride or dicyclohexylcarbodiimide) in the presence of organic base (for example, N-methylmorpholine, diisopropylethylamine or triethylamine) to give a compound of Formula IX which can be deprotected in an organic solvent (for example, methanol, ethanol, propanol, isopropylalcohol, tetrahydrofuran or ethyl acetate) under condition of deprotection (for example, hydrogenatically utilizing palladium an carbon or under catalytic hydrogen transfer conditions of ammonium formate and palladium on carbon) to give a compound of Formula X which can be reacted with a compound of Formula VI in an organic solvent (for example, acetonitrile, dimethylsulphoxide or dimethylformamide) in the presence of a base (for example, potassium carbonate, sodium carbonate or sodium bicarbonate) to give a compound of Formula XI.

Particular compounds according to the invention are shown here:
N-[(1α, 5 α, 6 α)-3-aza-bicyclo[3.1.0]hex-6-ylmethyl]-9H-Xanthene-9-carboxylic acid amide (Compound No. 2);
N-[(1 α, 5 α, 6 α)-3-aza-bicyclo[3.1.0]hex-6-yl)]-9H-Xanthene-9-carboxylic acid amide (Compound No. 6);
N-[(1 α, 5 α, 6 α)-3-(4-methyl-pent-3-enyl)-3-aza-bicyclo[3.1.0]hex-6-ylmethyl]-9H-Xanthene-9-carboxylic acid amide (Compound No. 8);
N-[(1 α, 5 α, 6 α)-3-(4-methyl-pent-3-enyl)-3-aza-bicyclo[3.1.0]hex-6-yl]- 9H-Xanthene-9-carboxylic acid amide (Compound No. 9);
N-[(1 α, 5 α, 6 α)-3-(2-(2,3-dihydro-benzofuran-5-yl)-ethyl)-3-aza-bicyclo[3.1.0]hex-6-ylmethyl]-9H-Xanthene-9-carboxylic acid amide (Compound No. 11).

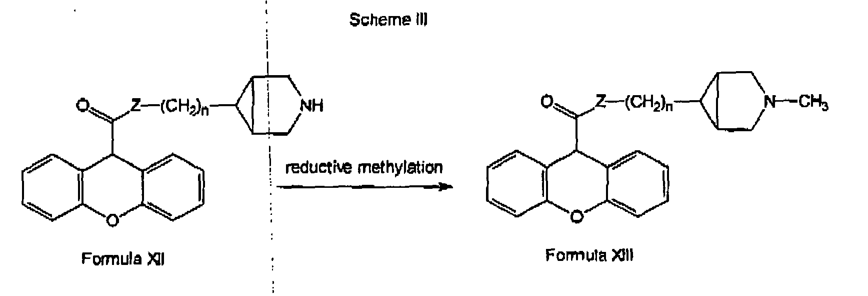

The compound of Formula XIII may be prepared, for examples, by the reaction sequence as shown in Scheme III. The compound of Formula XII (wherein Z is O or - NRₓ wherein Rₓ is the same as defined earlier and n is the same as defined earlier) undergoes reductive methylation to give a compound of Formula XIII.

The reductive methylation of a compound of Formula XII can be carried out in an organic solvent (for example, acetonitrile or dichloromethane) with formaldehyde in the presence of reducing agent (for example, sodium cyanoborohydride or sodium triacetoxy borohydride) to give a compound of Formula XIII.

Compounds according to the invention prepared following Scheme III are:
N-[(1α,5α,6α)-3-methyl-3-aza-bicyclo[3.1.0]hex-6-yl]-9H-Xanthene-9-carboxylic acid amide (Compound No. 3);
N-[(1α,5α,6α)-3-methyl-3-aza-bicyclo[3.1.0]hex-6-ylmethyl]-9H-Xanthene-9-carboxylic acid amide (Compound No. 4);
9H-Xanthene-9-carboxylic acid [(1α,5α,6α)-3-methyl-3-aza-bicyclo[3.1.0]hex-6-ylmethyl] ester (Compound Na. 7).

In the above scheme, where specific bases, condensing agents, protecting groups, deprotecting agents, solvents, catalysts, temperatures, etc. are mentioned, it is to be understood that other bases, condensing agents, protecting groups, deprotecting agents, solvents, catalysts, temperatures, etc. known to those skilled in the art may be used. Similarly, the reaction temperature and duration may be adjusted according to the desired needs.

Suitable salts of the compounds represented by the Formula I were prepared so as to solubilize the compound in aqueous medium for biological evaluations, as well as to be compatible with various dosage formulations and also to aid in the bioavailability of the compounds. Examples of such salts include pharmacologically acceptable salts such as inorganic acid salts (for example, hydrochloride, hydrobromide, sulphate, nitrate and phosphate), organic acid salts (for example, acetate, tartarate, citrate, fumarate, maleate, tolounesulphonate and methanesulphonate). When carboxyl groups are included in the Formula I as substituents, they may be present in the form of an alkaline or alkali metal salt (for example, sodium, potassium, calcium, magnesium, and the like). These salts may be prepared by various techniques, such as treating the compound with an equivalent amount of inorganic or organic, acid or base in a suitable solvent.

**Table 1**

| Compound No. | Structure | Compound No. | Structure |
|---|---|---|---|
| 1. | 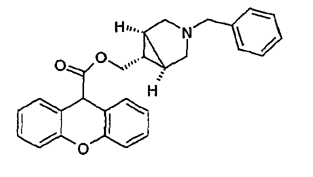 | 2. | 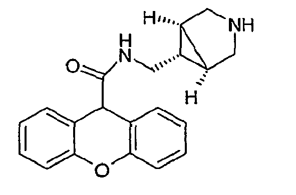 |
| 3. | 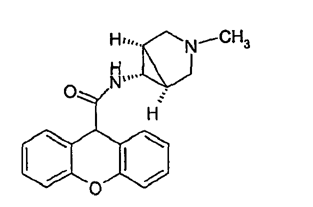 | 4. | 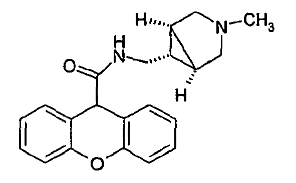 |
| 5. | 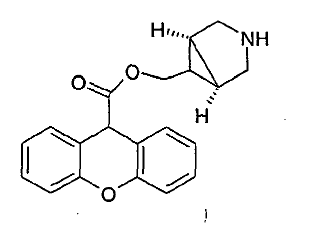 | 6. | 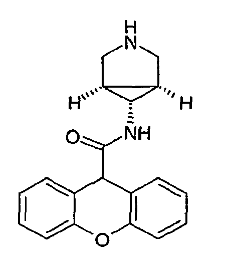 |
| 7. | 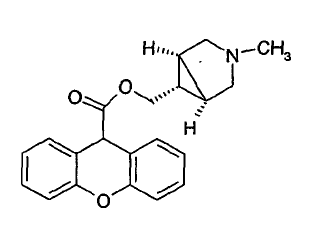 | 8. | 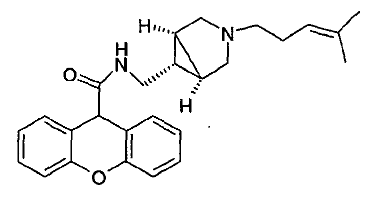 |
| 9. | 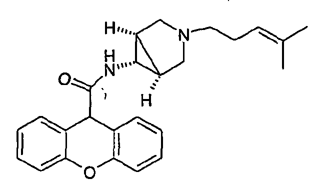 | 10. | 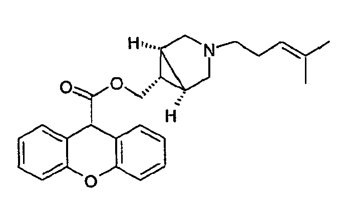 |
| 11. | 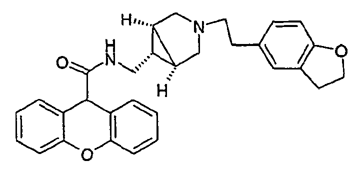 | | |

Because of their valuable pharmacological properties, the compounds described herein may be administered to an animal for treatment orally, or by a parenteral route. The pharmaceutical compositions described herein can be produced and administered in dosage units, each unit containing a certain amount of at least one compound described herein and/or at least one physiologically acceptable addition salt thereof. The dosage may be varied over extremely wide limits as the compounds are effective at low dosage levels and relatively free of toxicity. The compounds may be administered in the low micromolar concentration, which is therapeutically effective, and the dosage may be increased as desired up to the maximum dosage tolerated by the patient.

The compounds described herein can be produced and formulated as their enantiomers, diastereomers, N-Oxides, polymorphs, solvates and pharmaceutically acceptable salts, as well as metabolites having the same type of activity. Pharmaceutical compositions comprising the molecules of Formula I or metabolites, enantiomers, diastereomers, N-oxides, polymorphs, solvates or pharmaceutically acceptable salts thereof, in combination with pharmaceutically acceptable carrier and optionally included excipient can also be produced.

The examples mentioned below demonstrate general synthetic procedures, as well as specific preparations of particular compounds. The examples are provided to illustrate the details of the invention and do not limit the scope of the present invention.

### Examples

Various solvents, such as acetone, methanol, pyridine, ether, tetrahydrofuran, hexanes, and dichloromethane, were dried using various drying reagents according to procedures described in the literature. IR spectra were recorded as nujol mulls or a thin neat film on a Perkin Elmer Paragon instrument, Nuclear Magnetic Resonance (NMR) were recorded on a Varian XL-300 MHz instrument using tetramethylsilane as an internal standard.

### Synthesis of (1α,5α,6α)-6-hydroxymethyl-3-benzyl-3-aza-bicyclo[3.1.0]hexane

The compound was prepared by following the procedure described in *Synlett,* 1996, page 1097 by using N-phenylmaelamide.

### Synthesis of (1α,5α,6α)-6-(methylsulphonyloxy)methyl-3-benzyl-3-azabicyclo[3.1.0]hexane

To a solution of the compound (1α,5α,6α)-6-hydroxymethyl- 3-benzyl-3-azabicyclo[3.1.0]hexane (25 g, 123.2 mmol), triethylamine (35 ml, 246.4 mmol) in dichloromethane, was added 4-dimethyl amino pyridine (0.3 g, 2.5 mmol) followed by the addition of methane sulphonyl chloride (14.5 ml, 185 mmol) dropwise at 0-5°C. The reaction mixture was stirred at 25-30°C for approx. 15 hours. The reaction mixture was diluted with dichloromethane and washed with a saturated aqueous solution of sodium bicarbonate. The organic layer was separated, washed with water and brine solution, dried over anhydrous sodium sulphate and concentrated under reduced pressure to yield the title compound (74%).

### Synthesis of (1α, 5α, 6α)-6-aminomethyl-3-benzyl-3-azabicyclo[3.1.0]hexane

The title compound was prepared following the procedure as described in EP0413455.

### Synthesis of (1α, 5α, 6α)-6-amino-3-benzyl-3-azabicyclo[3.1.0]hexane

The title compound was prepared following the procedure as described in T.F. Braish. et. al. *Synlett.* 1996, 1100.

### Synthesis of N-[(1α, 5α, 6α)-3-benzyl-3-aza-bicyclo[3.1.0]hex-6-yl]-9H-Xanthene-9-carboxylic acid amide

A solution of 9H- xanthene-9-carboxylic acid (commercially available) (1.0 eq) and (1α, 5α, 6α)-6-amino-3-benzyl-3-aza-bicyclo[3.1.0]hexane (0.95 eq) in dimethylformamide was cooled to 0°C. To the resulting reaction mixture was added hydroxybenzotriazole (1 eq) and N-methylmorpholine (2eq). The reaction mixture was stirred for 30 minutes at 0°C followed by the addition of 1-(3-dimethylaminopropyl)-3-ethylcarbodiimide hydrochloride (1eq). The reaction mixture was again stirred for 1 hour at 0°C and thereafter it was stirred at room temperature for 24 hours. The reaction mixture was poured into water under stirring and extracted with ethylacetate. The solvent was evaporated under reduced pressure and the residue thus obtained was purified by column chromatography to furnish the title compound.

### Synthesis of N-[(1α,5α,6α)-3-benzyl-3-aza-bicyclo[3.1.0]hex-6-ylmethyl)-9H-xanthene-9-carboxylic acid amide

The title compound was prepared by following the procedure as described for the synthesis of N-[(1α,5α,6α)-3-benzyl-3-aza-bicyclo[3.1.0]hex-6-yl]-9H-Xanthene-9-carboxylic acid amide by using (1α, 5α, 6α)-6-aminomethyl-3-benzyl-3-azabicyclo[3.1.0]hexane in place of (1α, 5α, 6α)-6-amino-3-benzyl-3-azabicyclo[3.1.0]hexane

### SCHEME I PROCEDURE

### Example 1: Synthesis of 9H-Xanthene-9-carboxylic acid [(1α,5α,6α)-3-benzyl-3-azabicyclo[3.1.0]hex-6-ylmethyl] ester (Compound No.1)

To a solution of 9H-xanthene-9-carboxylic acid (1.1 eq) and (1α,5α,6α)-3-benzyl-6-methanesulphonyloxymethyl-3-aza-bicyclo[3.1.0]hexane (1.0 eq) in toluene, was added 1,8-diazabicyclo[5.4.0]undecane-4-ene (1 eq). The reaction mixture was refluxed for about 8 hours and then cooled to room temperature and stirred for overnight. The reaction mixture was quenched with sodium bicarbonate solution and toluene layer was separated. The organic layer was washed with water, brine and dried over anhydrous sodium sulphate. The organic layer was concentrated under reduced pressure. The residue thus obtained was purified by column chromatography to furnish the title compound (46%).
m.p: softening start at 85°C.
IR (KBr): 1733.7 cm⁻¹
¹H NMR (CDCl₃):δ 6.94-7.23 (m, 13H), 4.92 (s, 1H), 3.81-3.83 (m, 2H), 3.50 (s, 1H), 2.78-2.81 (m, 2H), 1.97-2.01 (m, 2H), 1.14-1.25 (m, 1H), 0.88-0.93 (m, 2H).
Mass (m/z): 412 (M⁺+1)

### Example 2: Synthesis of 9H-Xanthene-9-carboxylic acid [(1α, 5α, 6α)-1-(3-azabicyclo[3.1.0]hex-6-yl)methyl] ester (Compound No. 5)

To a solution of compound No. 1 (1.0 g) in dry methanol (25.0 ml), was added palladium on carbon (5%, 0.2 g) under nitrogen atmosphere followed by the addition of ammonium formate (0.8 g) under constant stirring. The reaction mixture was refluxed for half an hour under N₂ atmosphere. The reaction mixture was cooled to room temperature and filtered through hyflobed. The hyflobed was washed with methanol (75.0 ml), ethylacetate and water. The filtrate was concentrated under vacuum. The residue thus obtained was diluted with water and the pH of the resulting solution was adjusted to pH 14 with aqueous sodium hydroxide solution (10%). The compound was extracted with ethyl acetate and the organic layer was dried over anhydrous sodium sulphate and concentrated to give the title compound (80%).
IR (DCM): 1733.5 cm⁻¹
¹H NMR (CDCl₃):δ 6.99-7.23 (m, 8H), 4.93 (s, 1H), 3.82-3.91 (m, 2H), 2.93-2.96 (m, 2H), 2.25 (s, 2H), 1.18-1.35 (m, 3H).
Mass (m/z): 322 (M⁺+1).

### Example 3: Synthesis of 9H-Xanthene-9-carboxylic acid [(1α, 5α, 6α)-3-(4-methyl-pent-3-enyl)-3-aza-bicyclo[3.1.0]hex-6-ylmethyl] ester (Compound No. 10)

To a solution of compound No. 5 (1 mmol) in acetonitrile (5.0 ml), was added 5-bromo-2-methyl-pent-2-ene (1.2 mmol), potassium carbonate (8 mmol) and potassium iodide (2 mmol). The reaction mixture refluxed overnight. The reaction mixture was concentrated under reduced pressure and the residue thus obtained was taken in water and extracted with ethyl acetate. The organic layer was dried over anhydrous sodium sulphate and the solvent was evaporated under reduced pressure. The residue thus obtained was purified by column chromatography using ethyl acetate in hexane as eluent to furnish the title compound.
IR (DCM): 1738.8 cm⁻¹
¹H NMR (CDCl₃):δ 7.29-7.35 (m, 4H), 7.07-7.10 (m, 4H), 5.00-5.03 (m, 2H), 3.92-3.95 (m, 2H), 3.10 (m, 2H), 2.5 (m, 2H), 2.28-2.31 (m, 2H), 1.69 (s, 3H), 1.63 (s, 3H), 1.10-1.20 (m, 3H).
Mass (m/z): 404 (M⁺+1).

### SCHEME II PROCEDURE

### Example 4: Synthesis of N-[(1α, 5α, 6α)-(3-aza-bicyclo[3.1.0]hex-6-ylmethyl]-9H-Xanthene-9-carboxylic acid amide (Compound No. 2)

To a solution of N-*[(1*α,*5*α,*6*α*)*-3-benzyl-3-aza-bicyclo[3.1.0]hex-6-ylmethyl)-9H-Xanthene-9-carboxylic acid amide (1.0 g) in dry methanol (25.0 ml), was added palladium on carbon (5%, 0.2 g) under N₂ atmosphere followed by the addition of ammonium formate (0.8 g) under constant stirring. The reaction mixture was refluxed for half an hour under N₂ atmosphere. The reaction mixture was cooled to room temperature and filtered through hyflobed. The hyflobed was washed with methanol (75.0 ml), ethyl acetate (25.0 ml) and water. The filtrate was concentrated under vacuum. The residue thus obtained was diluted with water and pH of the resulting solution was adjusted to pH 14 with aqueous sodium hydroxide (10%)The compound was extracted with ethyl acetate and the ethyl acetate layer was washed with water and brine solution. The organic layer was dried over anhydrous sodium sulphate and concentrated to give the title compound (51%).
IR (KBr): 1641.0 cm⁻¹
¹H NMR (CDCl₃):δ 7.09-7.41 (m, 8H), 5.31 (brs, 1H), 4.88 (s, 1H), 3.03-3.07 (m, 2H), 2.73-2.83 (m, 4H), 1.16 (s, 2H), 0.57-0.62 (m, 1H).
Mass (m/z): 321 (M⁺+1).

The analog of [(1α,5α,6α)-3-aza-bicyclo[3.1.0]hex-6-ylmethyl]-9H-xanthene-9-carboxylic acid amide (Compound No. 2) described below, can be prepared by replacing N-[(1α,5α,6α)-3-benzyl-3-aza-bicyclo[3.1.0]hex-6-yl]-9H-Xanthene-9-carboxylic acid amide in place of N-[(1α,5α,6α)-3-benzyl-3-azabicyclo[3.1.0]hex-6-ylmethyl]- 9H-Xanthene-9-carboxylic acid amide.

### N-[(1α,5α,6α)-3-aza-bicyclo[3.1.0]hex-6-yl]-9H-xanthene-9-carboxylic acid amide (Compound No. 6)

m.p: 183-190°C
IR (KBr): 1645.6 cm⁻¹
¹H NMR (CDCl₃):δ 7.27-7.38 (m, 4H), 7.08-7.13 (m, 4H), 5.32 (brs, 1H), 4.85 (s, 1H), 3.04-3.08 (m, 2H), 2.80-2.84 (m, 2H), 2.33 (s, 1H), 0.88-0.90 (m, 2H).
Mass (m/z): 307 (M⁺+1).

### Example 5: Synthesis of N-[(1α, 5α, 6α)-3-(4-methyl-pent-3-enyl)-3-azabicyclo[3.1.0]hex-6-ylmethyl)-9H-xanthene-9-carboxylic acid amide (Compound No. 8)

To a solution of the compound No. 2 (1 mmol) in acetonitrile (5.0 ml), was added 5-bromo-2-methyl-pent-2-ene (1.2 mmol), potassium carbonate (8 mmol) and potassium iodide (2 mmol). The reaction mixture was refluxed for overnight. The reaction mixture was concentrated under reduced pressure and the residue thus obtained was taken in water and extracted with ethyl acetate. The organic layer was dried over anhydrous sodium sulphate and the solvent was evaporated under reduced pressure. The residue thus obtained was purified by column chromatography using ethylacetate in hexane as eluent to furnish the title compound (80%).
IR (KBr): 1639.9 cm⁻¹
¹H NMR (CDCl₃):δ 7.28-7.40 (m, 4H), 7.10-7.15 (m, 4H), 5.50 (brs, 1H), 5.00 (m, 1H), 4.87 (s, 1H), 3.03-3.07 (m, 2H), 2.64-2.66 (m, 4H), 2.33 (m, 2H), 1.68 (s, 3H), 1.62 (s, 3H), 1.33-1.37 (m, 2H), 0.86-0.88 (m, 1H).
Mass (m/z): 403 (M⁺+1).

The analog of N-[(1α, 5α, 6α)-3-(4-methyl-pent-3-enyl)-3-aza-bicyclo[3.1.0]hex-6-ylmethyl)-9H-Xanthene-9-carboxylic acid amide (Compound No. 8) described below, can be prepared by replacing 5-bromo-2-methyl-pent-2-ene with appropriate group.

### N-[(1α, 5α, 6α)-3-[2-(2,3-dihydro-benzofuran-5-yl)-ethyl]-3-aza-bicyclo[3.1.0]hex-6-ylmethyl}-9H-Xanthene-9-carboxylic acid amide(Compound No. 11)

m.p: 165°C
IR (KBr): 1642.2 cm⁻¹
¹H NMR (CDCl₃):δ 7.08-7.40 (m, 8H), 6.98 (s, 1H), 6.86-6.88 (m, 1H), 6.65-6.68 (m, H), 5.29-5.32 (m, 1H), 4.87 (s, 1H), 4.52 (t, J=9Hz, 2H), 3.15 (t, J=9Hz, 2H), 2.97-2.99 (m, 4H), 2.59-2.61 (m, 4H), 2.27-2.30 (m, 2H), 1.10-1.41 (m, 2H), 0.85-0.87 (m, 1H).
Mass (m/z): 467 (M⁺+1).

### Example 6: Synthesis of N-[(1α, 5α, 6α)-3-(4-methyl-pent-3-enyl)-3-azabicyclo[3.1.0]hex-6-yl]-9H-Xanthene-9-carboxylic acid amide (Compound No. 9)

The title compound was prepared by following the procedure as described for compound No. 8 by using compound No. 6 in place of compound No. 2 to furnish the title compound with 90% yield.
IR (KBr): 1648.8 cm⁻¹
¹H NMR (CDCl₃):δ 7.30-7.38 (m, 4H), 7.08-7.13 (m, 4H), 5.27 (brs, 1H), 5.01 (m, 1H), 4.85 (s, 1H), 3.08-3.11 (m, 2H), 2.35-2.40 (m, 4H), 2.06-2.09 (m, 2H), 1.65 (s, 3H), 1.57-1.60 (m, 3H), 1.33-1.36 (m, 2H), 0.86-0.90 (m, 1H).
Mass (m/z): 389 (M⁺+1).

### SCHEME III PROCEDURE:

### Example 7 Synthesis of 9H-Xanthene-9-carboxylic acid [(1α, 5α, 6α)-3-methyl-3-azabicyclo[3.1.0]hex-6-ylmethyl] ester (Compound No. 7)

To a solution of compound No. 5 (0.99 mmol) in acetonitrile (18.0 ml), formaldehyde (2.5 ml) and sodium cyanoborohydride (0.23 g) were added at room temperature and stirred for about 1 hour. Acetic acid (0.5 ml) was added to the reaction mixture and stirring was continued for 2 more hours at room temperature. Acetonitrile was evaporated off under reduced pressure and the residue was diluted with water (50.0 ml) and basified with aqueous sodium hydroxide. Extracted with ethyl acetate, washed with water, brine solution and dried over anhydrous sodium sulphate. The solvent was evaporated under reduced pressure and the residue thus obtained was purified by column chromatography using ethyl acetate in hexane as eluent.
m.p: softening start at 65°C
IR (KBr): 1734.6 cm⁻¹
¹H NMR (CDCl₃): δ 7.29-7.31 (m, 4H), 7.05-7.14 (m, 4H), 5.00 (s, 1H), 3.89-3.91 (m, 2H), 2.92-2.95 (m, 2H), 2.25-2.29 (m, 5H), 1.29-1.47 (m, 2H), 0.86-0.90 (m, 1H).
Mass (m/z): 336 (M⁺+1).

The analogues of 9H-Xanthene-9-carboxylic acid [(1α, 5α, 6α)-3-methyl-3-azabicyclo[3.1.0]hex-6-ylmethyl]ester (Compound No. 7) described below, can be prepared by replacing appropriate amine in place of Compound No. 5, respectively as applicable in each case.

N-[(1α, 5α, 6α)-3-methyl-3-aza-bicyclo[3.1.0]hex-6-yl]-9H-Xanthene-9-carboxylic acid amide (Compound No. 3);

N-[(1α, 5α, 6α)-3-methyl-3-aza-bicyclo[3.1.0]hex-6-ylmethyl]-9H-Xanthene-9-carboxylic acid amide (Compound No. 4).

### Biological Activity

### Radioligand Binding Assays:

The affinity of test compounds for M₂ and M₃ muscarinic receptor subtypes was determined by [³H]-N-methylscopolamine binding studies using rat heart and submandibular gland respectively as described by Moriya et al., (Life Sci., 1999,64(25):2351-2358) with minor modifications.

**Membrane preparation:** Submandibular glands and heart were isolated and placed in ice cold homogenising buffer (HEPES 20mM, 10mM EDTA, pH 7.4) immediately after sacrifice. The tissues were homogenised in 10 volumes of homogenising buffer and the homogenate was filtered through two layers of wet gauze and filtrate was centrifuged at 500g for 10min. The supernatant was subsequently centrifuged at 40,000g for 20 min. The pellet thus obtained was resuspended in assay buffer (HEPES 20 mM, EDTA 5mM, pH 7.4) and were stored at -70°C until the time of assay.

**Ligand binding assay:** The compounds were dissolved and diluted in DMSO. The membrane homogenates (150-250 µg protein) were incubated in 250 µl of assay volume (HEPES 20 mM, pH 7.4) at 24-25°C for 3h. Non-specific binding was determined in the presence of 1 µM atropine. The incubation was terminated by vacuum filtration over GF/B fiber filters(Wallac). The filters were then washed with ice cold 50mM Tris HCl buffer (pH 7.4). The filter mats were dried and bound radioactivity retained on filters was counted. The IC₅₀ & Kd were estimated by using the non-linear curve fitting program using G Pad Prism software. The value of inhibition constant Ki was calculated from competitive binding studies by using Cheng & Prusoff equation (Biochem Pharmacol, 1973, 22: 3099-3108), Ki = IC₅₀ /(1+L/Kd), where L is the concentration of [³H]NMS used in the particular experiment. pki is -log [Ki].

### Functional Experiments using isolated rat bladder:

### Methodology:

Animals were euthanized by overdose of thiopentone and whole bladder was isolated and removed rapidly and placed in ice cold Tyrode buffer with the following composition (mMol/L) NaCl 137; KCl 2.7; CaCl₂ 1.8; MgCl₂ 0.1; NaHCO₃ 11.9; NaH₂PO₄ 0.4; Glucose 5.55 and continuously gassed with 95% O₂ and 5 % CO₂.

The bladder was cut into longitudinal strips (3mm wide and 5-6 mm long) and mounted in 10 ml organ baths at 30° C, with one end connected to the base of the tissue holder and the other end connected through a force displacement transducer. Each tissue was maintained at a constant basal tension of 1 g and allowed to equilibrate for 1^{1/2} hour during which the Tyrode buffer was changed every 15-20 min. At the end of equilibration period the stabilization of the tissue contractile response was assessed with 1µmol/L of Carbachol till a reproducible response is obtained. Subsequently a cumulative concentration response curve to carbachol (10⁻⁹ mol/L to 3 X 10⁻⁴ mol/L) was obtained.
After several washes, once the baseline was achieved, cumulative concentration response curve was obtained in presence of NCE (NCE added 20 min. prior to the second cumulative response curve.
The contractile results were expressed as % of control E max. ED₅₀ values were calculated by fitting a non-linear regression curve (Graph Pad Prism). pKb values were calculated by the formula pKb = - log [(molar concentration of antagonist/ (dose ratio-1))] where, dose ratio = ED₅₀ in the presence of antagonist/ED₅₀ in the absence of antagonist.
The pKi values for the compounds were found to be in the range of 5-10 for both of the receptors.

While the present invention has been described in terms of its specific embodiments, certain modification and equivalents will be apparent to those skilled in the art and are intended to be included within the scope of the present invention.

## Claims

1. A compound selected from
9H-Xanthene-9-carboxylic acid [(1α, 5α, 6α)-3-benzyl]3-aza-bicyclo[3.1.0]hex-6-ylmethyl] ester (Compound No. 1);
N-[(1α, 5α, 6α)-3-aza-bicyclo[3.1.0]hex-6-ylmethyl]-9H-Xanthene-9-carboxylic acid amide (Compound No. 2);
N-[(1α, 5α, 6α)-3-methyl-3-aza-bicyclo[3.1.0]hex-6-yl]-9H-Xanthene-9-carboxylic acid amide (Compound No. 3);
N-[(1α, 5α, 6α)-3-methyl-3-aza-bicyclo[3.1.0]hex-6-ylmethyl]-9H-Xanthene-9-carboxylic acid amide (Compound No. 4);
9H-Xanthene-9-carboxylic acid [(1α, 5α, 6α))-3-aza-bicyclo[3.1.0]hex-6-ylmethyl] ester (Compound No. 5);
N-[(1α, 5α, 6α)-3-aza-bicyclo[3.1.0]hex-6-yl)]-9H-Xanthene-9-carboxylic acid amide (Compound No. 6);
9H-Xanthene-9-carboxylic acid [(1α, 5α, 6α)-3-methyl-3-aza-bicyclo[3.1.0]hex-6-ylmethyl] ester (Compound No. 7);
N-[(1α,5α,6α)-3-(4-methyl-pent-3-enyl)-3-aza-bicyclo[3.1.0]hex-6-ylmethyl]-9H-Xanthene-9-carboxylic acid amide (Compound No. 8);
N-[(1α, 5α, 6α)-3-(4-methyl-pent-3-enyl)-3-aza-bicyclo[3.1.0]hex-6-yl]- 9H-Xanthene-9-carboxylic acid amide (Compound No. 9);
9H-Xanthene-9-carboxylic acid [(1α, 5α, 6α)-3-(4-methyl-pent-3-enyl)-3-azabicyclo[3.1.0]hex-6-ylmethyl] ester (Compound No. 10);
N-[(1α,5α,6α)-3-[(2-(2,3-dihydro-benzofuran-5-yl)-ethyl)-3-aza-bicyclo[3.1.0]hex-6-ylmethyl]-9H-Xanthene-9-carboxylic acid amide (Compound No. 11);
and their pharmaceutically acceptable salts, pharmaceutically acceptable solvates, enantiomers diastereomers, N-oxides or polymorphs.

2. A pharmaceutical composition comprising therapeutically effective amount of a compound as defined in claim 1 together with pharmaceutically acceptable carrier, excipients or diluents.

3. A compound as defined in claim 1 for treatment or prophylaxis of an animal or a human suffering from a disease or disorder of the respiratory, urinary and gastrointestinal systems, wherein the disease or disorder is mediated through the muscarinic receptors.

4. A compound according to claim 3 wherein the disease or disorder is urinary in continence, lower urinary tract symptoms (LUTS), bronchial asthma, chronic obstructive pulmonary disease (COPD), pulmonary fibrosis, irritable bowel syndrome, obesity, diabetes, and gastrointestinal hyperkinesis.

5. A pharmaceutical composition according to claim 2 for treatment or prophylaxis of an animal or a human suffering from a disease or disorder of the respiratory, urinary, and gastrointestinal systems, wherein the disease or disorder is mediated through the muscarinic receptors.

6. A composition according to claim 5 wherein the disease or disorder is urinary incontinence, lower urinary tract symptoms (LUTS), bronchial asthma, chronic obstructive pulmonary disease (COPD), pulmonary fibrosis, irritable based syndrome, obesity, diabetes and gastrointestinal tract hyperkinesis.

7. The method of preparing a compound of Formula VII 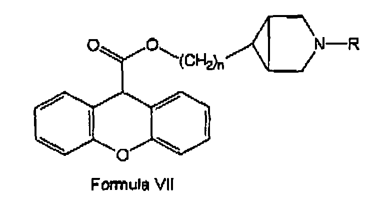 and its pharmaceutically acceptable salts, pharmaceutically acceptable solvates, enantiomers, diastereomers, N-oxides or polymorphs, wherein
R is heteroarylalkyl or alkenyl, and
n is an integer from 0-4
said method comprising:
a. condensing a compound of Formula II with a compound of Formula III (wherein L is a leaving group (for example, mesyl or tosyl, P is a protecting group (for example, aralkyl and n is the same as defined earlier) 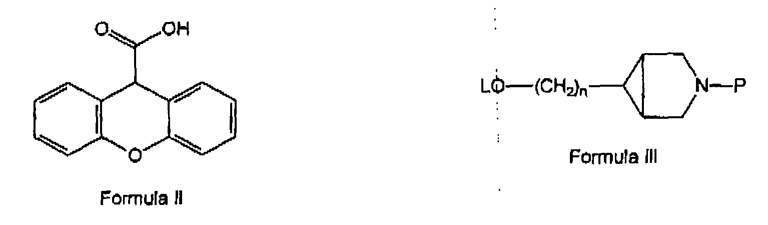 to give a compound of Formula IV, 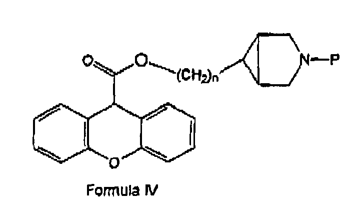
b. deprotecting the compound of Formula IV to give a compound of Formula V, and 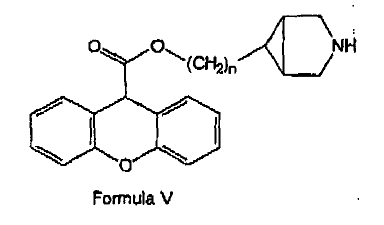
c. reacting the compound of Formula V with a compound of Formula VI,
(R-hal) Formula VI
to give a compound of Formula VII

8. The method of claim 7, wherein the condensation of a compound of Formula II with a compound of Formula III to give a compound of Formula IV is carried out with a condensing agent selected from 1,8-diazabicyclo[5.4.0]undecen-7-ene or 1,4-diazabicyclo [2.2.2]octane.

9. A method of preparing a compound of Formula XI, 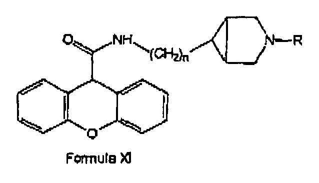 its pharmaceutically acceptable salts, pharmaceutically acceptable solvates, enantiomers, diastereomers, N-oxides or polymorphs, wherein
R is heteroarylalkyl, or alkenyl; and
n is an integer from 0-4,
said method comprising:
a. condensing a compound of Formula II with a compound of Formula VIII (wherein n is the same as defined earlier and P is a protecting group (for example, aralkyl). 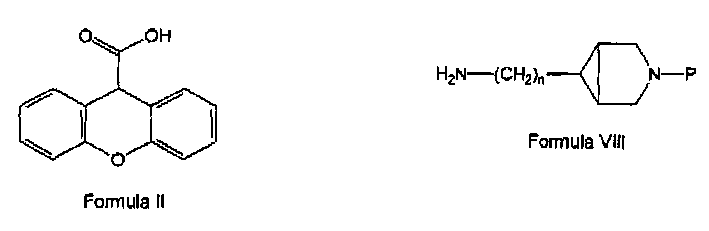 to give a compound of Formula IX, 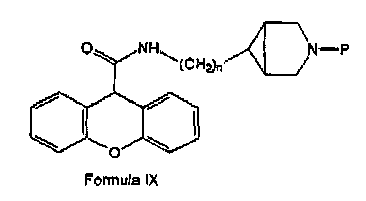
b. deprotecting the compound of Formula IX to give a compound X, and 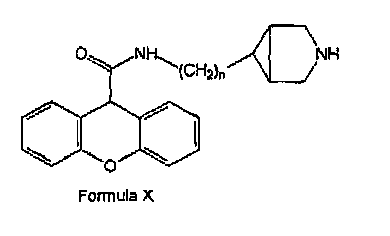
c. reacting the compound of Formula X with a compound of Formula VI,
(R-hal) Formula VI
to give a compound of Formula XI.

10. A method of preparing a compound of Formula XIII 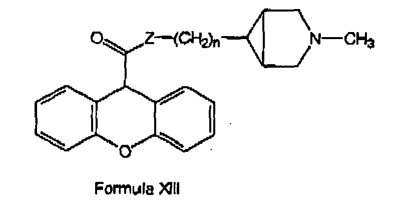 and its pharmaceutically acceptable salts, pharmaceutically acceptable solvates, enantiomers, diastereomers, N-oxide or polymorphs, wherein
Z is oxygen, or -NRx (wherein Rx is selected from hydrogen, lower (C₁₋₆) alkyl, and aralkyl); and
n is an integer from 0-4, in which compound of Formula XII 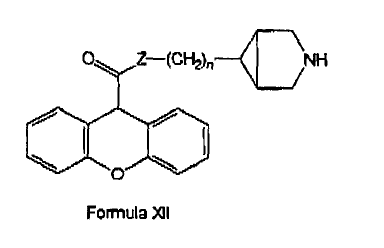 undergoes reductive methylation to give a compound of Formula XIII.

## Patentansprüche

1. Verbindung, ausgewählt aus
9H-Xanthen-9-carbonsäure-[(1α,5α,6α)-3-benzyl-3-azabicyclo[3.1.0]hex-6-ylmethyl]-ester (Verbindung Nr. 1),
N-[(1α,5α,6α)-3-Azabicyclo[3.1.0]hex-6-ylmethyl]-9H-xanthen-9-carboxamid (Verbindung Nr. 2),
N-[(1α,5α,6α)-3-Methyl-3-azabicyclo[3.1.0]hex-6-yl]-9H-xanthen-9-carboxamid (Verbindung Nr. 3),
N-[(1α,5α,6α)-3-Methyl-3-azabicyclo[3.1.0]hex-6-ylmethyl]-9H-xanthen-9-carboxamid (Verbindung Nr. 4),
9H-Xanthen-9-carbonsäure-[(1α,5α,6α))-3-azabicyclo[3.1.0]hex-6-ylmethyl]-ester (Verbindung Nr. 5),
N-[(1α,5α,6α)-3-Azabicyclo[3.1.0]hex-6-yl)]-9H-Xanthen-9-carboxamid (Verbindung Nr. 6),
9H-Xanthen-9-carbonsäure-[(1α,5α,6α)-3-methyl-3-azabicyclo[3.1.0]hex-6-ylmethyl]-ester (Verbindung Nr. 7),
N-[(1α,5α,6α)-3-(4-Methylpent-3-enyl)-3-azabicyclo[3.1.0]hex-6-ylmethyI]-9H-Xanthen-9-carboxamid (Verbindung Nr. 8),
N-[(1α,5α,6α)-3-(4-Methylpent-3-enyl)-3-azabicyclo[3.1.0]hex-6-yl]-9H-xanthen-9-carboxamid (Verbindung Nr. 9),
9H-Xanthen-9-carbonsäure-[(1α,5α,6α)-3-(4-methyl-pent-3-enyl)-3-azabicycIo[3.1.0]hex-6-ylmethyl]-ester (Verbindung Nr. 10),
N-[(1α,5α,6α)-3-[(2-(2,3-Dihydrobenzofuran-5-yl)-ethyl)-3-azabicyclo[3.1.0]hex-6-ylmethyl]-9H-xanthen-9-carboxamid (Verbindung Nr. 11)
und ihre pharmazeutisch zulässigen Salze, pharmazeutisch zulässigen Solvate, Enantiomere, Diastereomere, N-Oxide
oder Polymorphe.

2. Pharmazeutische Zusammensetzung mit einer therapeutisch wirksamen Menge einer Verbindung gemäß Anspruch 1 zusammen mit pharmazeutisch zulässigen Trägern, Hilfsstoffen oder Verdünnungsmitteln.

3. Verbindung nach Anspruch 1 zur Behandlung oder Vorbeugung bei einem Tier oder einem Menschen, welche an einer Erkrankung oder Störung des Atmungs-, Harn- und Magen-Darm-Systems leiden, wobei die Erkrankung oder Störung durch die Muscarinrezeptoren vermittelt wird.

4. Verbindung nach Anspruch 3, wobei die Erkrankung oder Störung Harninkontinenz, Syndrom der unteren Harnwege (LUTS), Bronchialasthma, chronisch obstruktive Lungenerkrankung (COPD), Lungenfibrose, Reizdarmsyndrom, Fettleibigkeit, Diabetes und Magen-Darm-Hyperkinese ist.

5. Pharmazeutische Zusammensetzung nach Anspruch 2 zur Behandlung oder Vorbeugung bei einem Tier oder einem Menschen, welche an einer Erkrankung oder Störung des Atmungs-, Harn- und Magen-Darm-Systems leiden, wobei die Erkrankung oder Störung durch die Muscarinrezeptoren vermittelt wird.

6. Zusammensetzung nach Anspruch 5, wobei die Erkrankung oder Störung Harninkontinenz, Syndrom der unteren Harnwege (LUTS), Bronchialasthma, chronisch obstruktive Lungenerkrankung (COPD), Lungenfibrose, Reizdarmsyndrom, Fettleibigkeit, Diabetes und Magen-Darm-Hyperkinese ist.

7. Verfahren zur Herstellung einer Verbindung der Formel VII 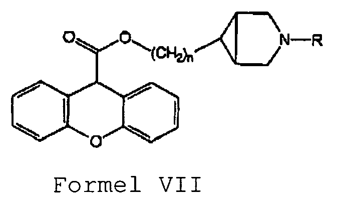 und ihrer pharmazeutisch zulässigen Salze, pharmazeutisch zulässigen Solvate, Enantiomere, Diastereomere, N-Oxide oder Polymorphe, wobei
R Heteroarylalkyl oder Alkenyl und
n eine ganze Zahl von 0 bis 4 ist, wobei das Verfahren
a. die Kondensation einer Verbindung der Formel II mit einer Verbindung der Formel III, worin L eine abspaltende Gruppe (z.B. Mesyl oder Tosyl), P eine Schutzgruppe (z.B. Aralkyl) und n dasselbe oben definiert ist 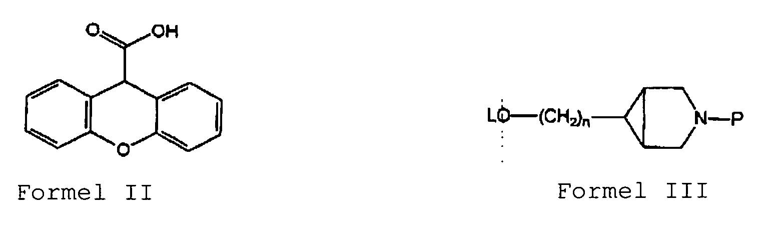 zur Bildung einer Verbindung der Formel IV 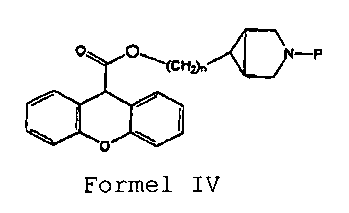
b. das Abspalten der Schutzgruppe von der Verbindung der Formel IV zur Bildung einer Verbindung der Formel V, und 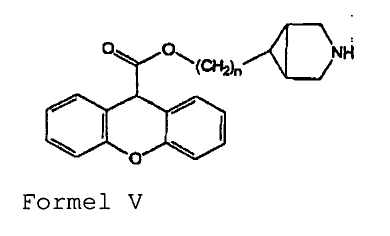
c. die Umsetzung der Verbindung der Formel V mit einer Verbindung der Formel VI
(R-hal) Formel VI
zur Bildung einer Verbindung der Formel VII umfasst.

8. Verfahren nach Anspruch 7, wobei die Kondensation einer Verbindung der Formel II mit einer Verbindung der Formel III zur Bildung einer Verbindung der Formel IV mit einem Kondensationsmittel erfolgt, das aus 1,8-Diazabicyclo[5.4.0]undec-7-en oder 1,4-Diazabicyclo-[2.2.2]octan ausgewählt ist.

9. Verfahren zur Herstellung einer Verbindung der Formel XI, 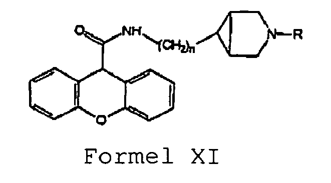 ihrer pharmazeutisch zulässigen Salze, pharmazeutisch zulässigen Solvate, Enantiomere, Diastereomere, N-Oxide oder Polymorphe, worin
R Heteroarylalkyl oder Alkenyl und
n eine ganze Zahl von 0 bis 4 ist, wobei das Verfahren
a. die Kondensation einer Verbindung der Formel II mit einer Verbindung der Formel VIII (wobei n dasselbe wie oben definiert und P eine Schutzgruppe, zum Beispiel Aryalkyl, ist) 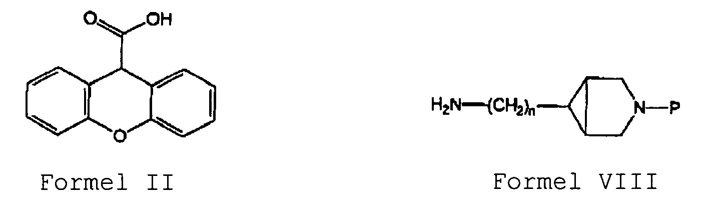 zur Bildung einer Verbindung der Formel IX, 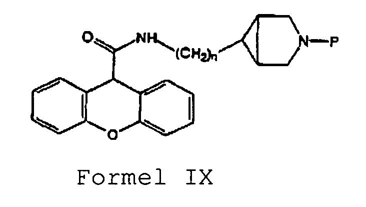
b. das Abspalten der Schutzgruppe von der Verbindung der Formel IX zur Bildung einer Verbindung X und 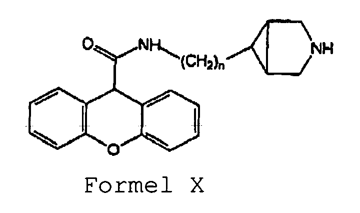
c. das Umsetzen der Verbindung der Formel X mit einer Verbindung der Formel VI
(R-hal) Formel VI
zur Bildung einer Verbindung der Formel XI umfasst.

10. Verfahren zur Herstellung einer Verbindung der Formel XIII 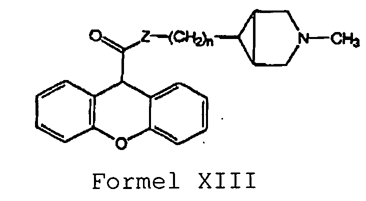 und ihrer pharmazeutisch zulässigen Salze, pharmazeutisch zulässigen Solvate, Enantiomere, Diastereomere, N-Oxide oder Polymorphe, worin
Z Sauerstoff oder -NRₓ ist (wobei Rₓ aus Wasserstoff, Niedrigalkyl (C₁₋₆) und Arylalkyl ausgewählt ist) und
n eine ganze Zahl von 0 bis 4 ist, wobei die Verbindung der Formel XII 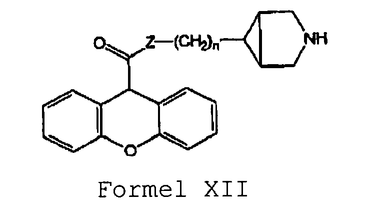 einer reduktiven Methylierung zur Bildung einer Verbindung der Formel XIII unterworfen wird.

## Revendications

1. Composé choisi parmi
[(1α,5α,6α)-3-benzyl-3-aza-bicyclo[3.1.0]hex-6-ylméthyl]ester d'aci de 9H-xanthène-9-carboxylique (Composé n° 1 ) ;
amide d'acide N-[(1α, 5α, 6α)-3-aza-bicyclo[3.1.0]hex-6-ylméthyl]-9H-xanthène-9-carboxylique (Composé n° 2) ;
amide d'acide N-[(1α, 5α, 6α)-3-méthyl-3-aza-bicyclo[3.1.0]hex-6-yl]-9H-xanthène-9-carboxylique (Composé n° 3) ;
amide d'acide N-[(1α, 5α, 6α)-3-méthyl-3-aza-bicyclo[3.1.0]hex-6-ylméthyl]-9H-xanthène-9-carboxylique (Composé n° 4) ;
[(1α, 5α, 6α)-3-aza-bicyclo[3.1.0]hex-6-ylméthyl]ester d'acide 9H-xanthène-9-carboxylique (Composé n° 5) ;
amide d'acide N-[(1α, 5α, 6α)-3-aza-bicyclo[3.1.0]hex-6-yl)]-9H-xanthène-9-carboxylique (Composé n° 6) ;
[(1α,5α,6α)-3-méthyl-3-aza-bicyclo[3.1.0]hex-6-ylméthyl]ester d'acide 9H-xanthène-9-carboxylique (Composé n° 7) ;
amide d'acide N-[(1α, 5αa, 6α)-3-(4-méthyl-pent-3-ényl)-3-aza-bicyclo[3.1.0]hex-6-ylméthyl]-9H-xanthène-9-carboxylique (Composé n° 8) ;
amide d'acide N-[(1α, 5α, 6α)-3-(4-méthyl-pent-3-ényl)-3-azabicyclo[3.1.0]hex-6-yl]- 9H-xanthène- 9-carboxylique (Composé n° 9) ;
[(1α,5α,6α)-3-(4-méthyl-pent-3-ényl)-3-aza-bicyclo[3.1.0]hex-6-ylméthyl]ester d'acide 9H-xanthène-9-carboxylique (Composé n° 10) ;
amide d'acide N-[(1α, 5α, 6α)-3-[(2-(2,3-dihydro-benzofuran-5-yl)-éthyl)-3-aza-bicyclo[3.1.0]hex-6-ylméthyl]-9H-xanthène-9-carboxylique (Composé n° 11) ;
et leurs sels pharmaceutiquement acceptables, solvates pharmaceutiquement acceptables, énantiomères, diastéréomères, N-oxydes ou polymorphes.

2. Composition pharmaceutique comprenant une quantité thérapeutiquement efficace d'un composé tel que défini dans la revendication 1 ainsi qu'un support, des excipients ou diluants pharmaceutiquement acceptables.

3. Composé selon la revendication 1 pour le traitement ou la prophylaxie d'un animal ou d'un être humain souffrant d'une maladie ou d'un trouble des systèmes respiratoire, urinaire et gastro-intestinal, la maladie ou le trouble étant médié par les récepteurs muscariniques.

4. Composé selon la revendication 3 dans laquelle la maladie ou le trouble est une incontinence urinaire, des symptômes du bas appareil urinaire (SBAU), un asthme bronchique, des troubles pulmonaires obstructifs chroniques (TPOC), une fibrose pulmonaire, un syndrome de l'intestin irritable, une obésité, un diabète ou une hyperkinésie gastro-intestinale.

5. Composition pharmaceutique selon la revendication 2 pour le traitement ou la prophylaxie d'un animal ou d'un être humain souffrant d'une maladie ou d'un trouble des systèmes respiratoire, urinaire et gastro-intestinal, la maladie ou le trouble étant médié par les récepteurs muscariniques.

6. Composition pharmaceutique selon la revendication 5, dans laquelle la maladie ou le trouble est une incontinence urinaire, des symptômes du bas appareil urinaire (SBAU), un asthme bronchique, des troubles pulmonaires obstructifs chroniques (TPOC), une fibrose pulmonaire, un syndrome de l'intestin irritable, une obésité, un diabète ou une hyperkinésie gastro-intestinale.

7. Procédé de préparation d'un composé de Formule VII 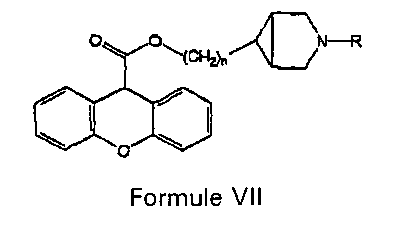 et ses sels pharmaceutiquement acceptables, solvates pharmaceutiquement acceptables, énantiomères, diastéréomères, N-oxydes ou polymorphes, dans laquelle
R est un hétéroarylalkyle ou un alkényle, et
n est un nombre entier de 0 à 4
ledit procédé consistant à :
a. condenser un composé de Formule II avec un composé de Formule III (où L est un groupe partant (par exemple mésyle ou tosyle), P est un groupe de protection (par exemple aralkyle) et n est le même que défini plus haut) 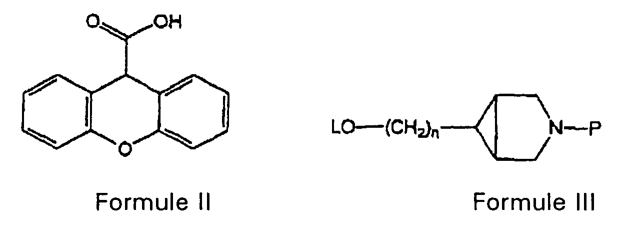 pour donner un composé de Formule IV, 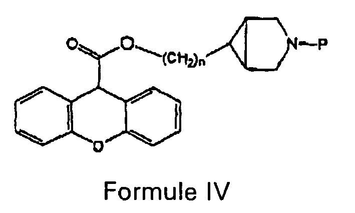
b. déprotéger le composé de Formule IV pour donner un composé de Formule V, et 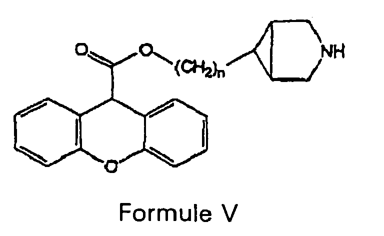
c. faire réagir le composé de Formule V avec un composé de Formule VI,
(R-hal) Formule VI
pour donner un composé de Formule VII.

8. Procédé selon la revendication 7, dans lequel la condensation d'un composé de Formule II avec un composé de Formule III pour donner un composé de Formule IV est réalisée avec un agent condensant choisi parmi le 1,8-diazabicyclo[5.4.0]undécén-7-ène ou le 1,4-diazabicyclo [2.2.2]octane.

9. Procédé de préparation d'un composé de Formule XI, 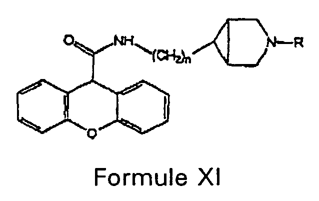 ses sels pharmaceutiquement acceptables, solvates pharmaceutiquement acceptables, énantiomères, diastéréomères, N-oxydes ou polymorphes, dans laquelle
R est un hétéroarylalkyle ou un alkényle ; et
n est un nombre entier de 0 à 4
ledit procédé consistant à :
a. condenser un composé de Formule II avec un composé de Formule VIII (où n est le même que défini plus haut et P est un groupe de protection (par exemple aralkyle). 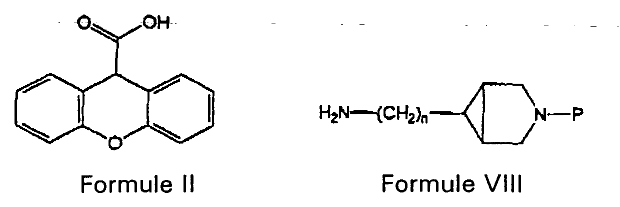 pour donner un composé de Formule IX, 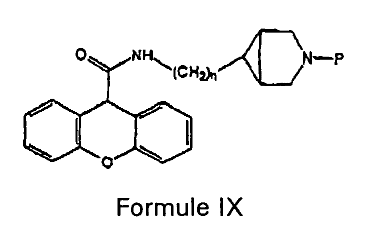
b. déprotéger le composé de Formule IX pour donner un composé X, et 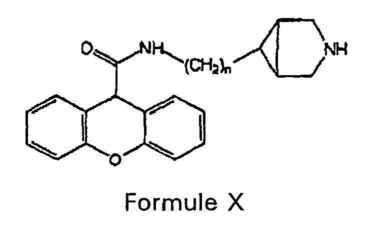
c. faire réagir le composé de Formule X avec un composé de Formule VI,
(R-hal) Formule VI
pour donner un composé de Formule XI.

10. Procédé de préparation d'un composé de Formule XIII, 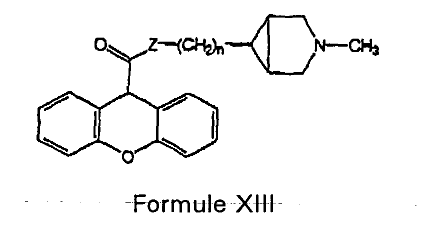 et ses sels pharmaceutiquement acceptables, solvates pharmaceutiquement acceptables, énantiomères, diastéréomères, N-oxydes ou polymorphes, dans laquelle
Z est un oxygène, ou -NRx (où Rx est choisi parmi un hydrogène, un alkyle inférieur en C₁₋₆ et un aralkyle) ; et
n est un nombre entier de 0 à 4, dans lequel le composé de Formule XII 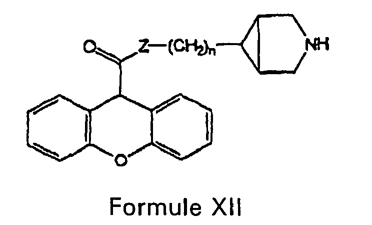 subit une méthylation réductrice pour donner un composé de Formule XIII.
